# EUROPEAN PATENT APPLICATION

(11) **EP 4 768 894 A1**
(43) Date of publication of application: **01.07.2026**
(21) Application number: 25226425.4
(22) Date of filing: 22.12.2025
(51) Int. Cl.: G01N 21/15, G01N 21/3504, G01N 33/00

(54) **PROTECTIVE COVER FOR OPTICAL GAS SENSOR AND OPTICAL GAS SENSOR DEVICE**

(30) Priority: 24.12.2024 JP 2024227337
(71) Applicant: Mitsumi Electric Co., Ltd., Tama-Shi, Tokyo 206-8567 (JP)
(72) Inventor: TAJIMA, Kazuki, Tokyo, 206-8567 (JP); OKAMOTO, Mitsuhiro, Tokyo, 206-8567 (JP)
(74) Representative: Epping - Hermann - Fischer

(57) **Abstract**

A protective cover (20) is for covering an optical gas sensor (10). The gas sensor includes: a casing body (11) that has a hollow part (13) into which a gas is introduced and a gas intake port (111, 112) that communicates with the hollow part; and a light source (2) and a light receiver (4) disposed to face each other with the hollow part in-between. The gas sensor is mounted on a board (6) and detects a gas concentration inside the hollow part, based on a detection signal of the light receiver that receives light emitted by the light source. The protective cover is box-shaped and has a wall. One side of the box-shaped protective cover is open. At least part of the wall has an opening (21A) provided with a filter (23). At an open edge of the one side, the protective cover has at least a pair of fitting pieces (22) to be fitted to fitting portions (6a) of the board.

## Description

### BACKGROUND OF THE INVENTION

### TECHNICAL FIELD

The present invention relates to a protective cover for protecting an optical gas sensor from dust and droplets. For example, the present invention is effectively applicable to a protective cover for an optical gas sensor mounted on a board.

### DESCRIPTION OF RELATED ART

Gas sensors may need to be protected from dust and droplets depending on the environment in which the sensors are installed. For example, Japanese Unexamined Patent Application Publication No. 2021-60217 discloses a gas sensor with a protective cover.

The protective cover for the gas sensor described in JP 2021-60217A consists of an inner protective cover and an outer protective cover. The inner and outer protective covers are attached to cover the tip end of a long planar-shaped sensor element. The sensor element is attached to the wall of a pipe, through which a detection target gas flows, such that the tip end of the sensor protrudes inside the pipe. The gas sensor described in JP 2021-60217A detects the concentration of gases, such as NOx in automobile exhaust gases. The sensor element of the gas sensor has a structure in which a plurality of oxygen-ion-conductive solid electrolyte layers made of zirconia (ZrO₂) or the like are laminated. The gas sensor may be installed in an environment of relatively high temperature.

A known gas sensor uses a non-dispersive infrared (NDIR) absorption method. The NDIR gas sensor utilizes the property of detection target gases each of which absorbs a specific infrared wavelength. The NDIR gas sensor emits infrared rays to a detection target gas, detects the absorbed wavelength and the amount of the absorbed wavelength, and thereby measures the concentration of the detection target gas. For example, the NDIR gas sensor includes an infrared light emitter and an infrared light receiver and detects the concentration of the detection target gas in an optical path between the light emitter and the light receiver.

An optical gas sensor takes in a detection target gas and may be mounted on a board on which electronic components are mounted. Such a gas sensor is disclosed in, for example, Japanese Unexamined Patent Application Publication No. 2023-162821.

Since the protective cover of JP 2021-60217A includes the inner and outer protective covers, the protective cover has many parts. The gas sensor of JP 2021-60217A and the light absorption gas sensor of JP 2023-162821A detect gases by different methods and have different structures. Therefore, the protective cover of JP 2021-60217A cannot be directly applied to the light absorption gas sensor of JP 2023-162821A.

For a light absorption gas sensor, a user may wish to attach a protective cover to the sensor that has already been installed and in operation. Since the gas sensor of JP 2021-60217A is attached to the pipe through which engine exhaust gases flow, it is difficult to attach a protective cover to the sensor having already been installed and in operation. Further, JP 2021-60217A does not describe such an issue.

The present invention has been conceived in view of the above issue. An object of the present invention is to provide a protective cover suitable for an optical gas sensor mounted on a board.

Another object of the present invention is to provide a protective cover that can be attached later for an optical gas sensor having already been installed and that protects the sensor from dust and droplets.

### SUMMARY OF THE INVENTION

To solve the above problem, according to the present invention, there is provided a protective cover to be mounted to cover an optical gas sensor, the optical gas sensor including: a casing body that has a hollow part into which a detection target gas is introduced and a gas intake port that communicates with the hollow part; and a light source and a light receiver disposed to face each other with the hollow part in-between, the optical gas sensor being mounted on a board and configured to detect a gas concentration inside the hollow part, based on a detection signal of the light receiver that receives light emitted by the light source, wherein: the protective cover is box-shaped and has a wall, one side of the box-shaped protective cover is open, at least part of the wall has an opening provided with a filter, and at an open edge of the one side, the protective cover has at least a pair of fitting pieces to be fitted to fitting portions of the board.

### ADVANTAGEOUS EFFECTS OF INVENTION

According to the present invention, a protective cover can protect an optical gas sensor mounted on a board from dust and droplets. Further, the protective cover can be attached to an optical gas sensor having already been installed.

### BRIEF DESCRIPTION OF DRAWINGS

The accompanying drawings are not intended as a definition of the limits of the invention but illustrate embodiments of the invention, and together with the general description given above and the detailed description of the embodiments given below, serve to explain the principles of the invention, wherein:
FIG. 1 is a perspective view of an NDIR optical gas sensor as an example of a protection target sensor to which the protective cover of the present invention is attached, wherein the external view and the internal structure of the NDIR optical gas sensor is shown;
FIG. 2 is an exploded perspective view of an example of upper and lower parts constituting a casing body (case) of the optical gas sensor shown in FIG. 1;
FIG. 3A is a bottom view of the upper part constituting the casing body of the optical gas sensor in FIG. 1, wherein the upper part is viewed from below;
FIG. 3B is a diagram for explaining reflection of infrared rays in a light guide;
FIG. 4 is a perspective view of a protective cover as an embodiment of the present invention and an NDIR optical gas sensor as an example;
FIG. 5 is a perspective view of the protective cover in the embodiment mounted on the board to cover the optical gas sensor mounted on the board;
FIG. 6 is a perspective view of another example of the protective cover in the embodiment; and
FIG. 7 is a plan view of the protective cover in the embodiment.

### DETAILED DESCRIPTION

Hereinafter, an embodiment of a protective cover for an optical gas sensor and an optical gas sensor device using the protective cover according to the present invention will be described in detail.

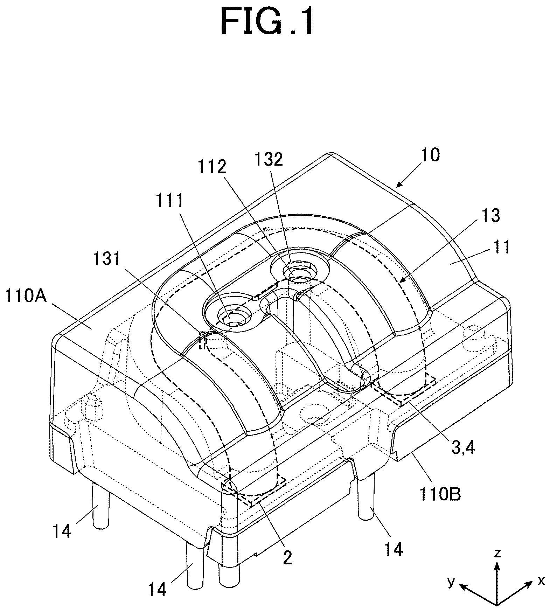

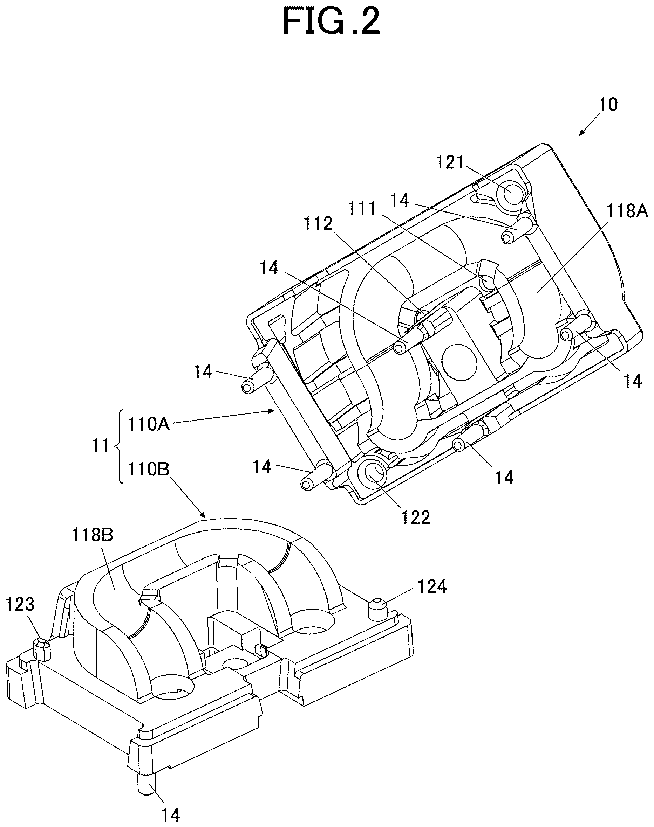

FIG. 1 shows a schematic configuration of an NDIR optical gas sensor as an example of a protection target sensor to which the protective cover of the present invention is attached. FIG. 2 shows an exploded perspective view of an example of upper and lower parts constituting a casing body (case) of the optical gas sensor in FIG. 1.

An optical gas sensor (hereinafter referred to as gas sensor) 10 includes a casing body 11. As shown in FIG. 2, the casing body 11 consists of an upper part 110A and a lower part 110B. The upper part 110A and the lower part 110B are made of synthetic resin, for example, and are joined together to form a unitary body, as shown in FIG. 1.

Inside the casing body 11, a pipe-shaped hollow part is formed. Into the hollow part, a detection target gas can be introduced. Both ends of the hollow part face downward (face in the -z direction). At the opening at one end of the hollow part, a light source 2 is disposed. At the opening at the other end of the hollow part, an optical filter 3 and a light receiver 4 are disposed. Thus, the casing body 11 is mounted on the board 6 so as to cover (house) the light source 2, the optical filter 3, and the light receiver 4. The detection target gas can be introduced into the hollow part in the casing body 11 through gas intake ports 111 and 112 formed in the casing body 11.

Specifically, as shown in FIG. 2, pins 123 and 124 are formed on the top surface of the lower part 110B of the casing body 11 for joining. The pins 123 and 124 for joining are projections extending in the +z direction. At the positions corresponding to the pins 123 and 124 on the bottom surface of the upper part 110A, recesses 121 and 122 having the same diameter as the projections are formed. The pins 123 and 124 for joining are fitted into the recesses 121 and 122 of the upper part 110A, so that the upper part 110A and the lower part 110B of the casing body 11 are aligned with each other and jointed as one body.

The casing body 11 includes a light guide 13. The light guide 13 is the hollow part into which the detection target gas is introduced, as shown in FIG. 1. The light guide 13 has a three-dimensionally twisted shape. The cross section of the light guide 13 in the direction perpendicular to its central axis has a round shape. Each section along the central axis has a uniform diameter. As shown in FIG. 2, the upper part 110A has an upper half-pipe part 118A that is a recess forming the light guide 13; and the lower part 110B has a lower half-pipe part 118B that is a recess forming the light guide 13. The half-pipe parts 118A and 118B are joined to form the pipe-shaped light guide 13. Thus, the casing body 11 consists of the upper part 110A and the lower part 110B, which are formed by dividing the light guide 13 into two parts (upper and lower parts) by a plane along the axial direction of the light guide 13.

The casing body 11 has positioning pins 14 that project downward.

Both ends of the light guide 13 are open. As shown in FIG. 1, the light source 2 is disposed to face one opening of the light guide 13; and the optical filter 3 and light receiver 4 are disposed to face the other opening of the light guide 13.

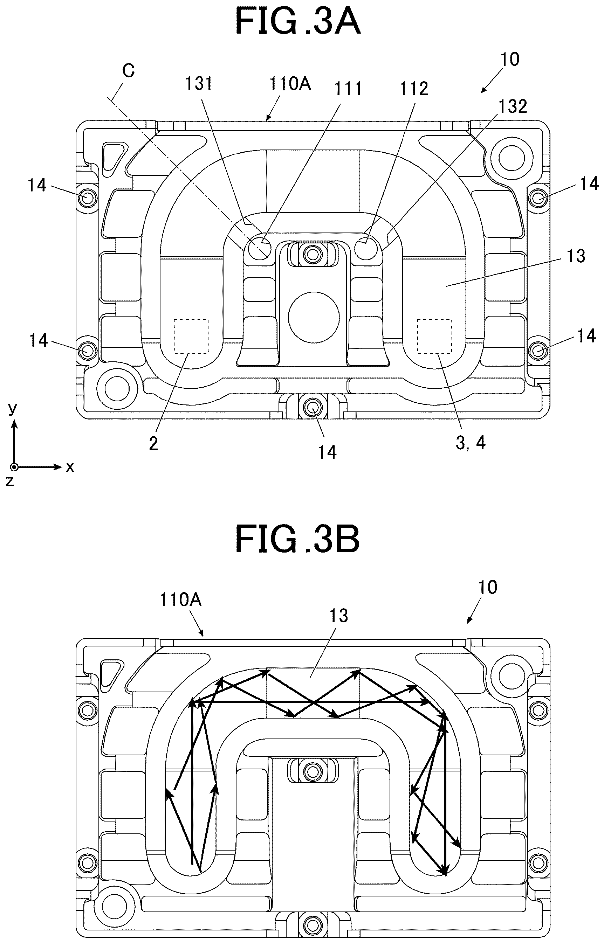

The light guide 13 is substantially U-shaped in a top view, as shown in FIG. 3A. The inner surface of the light guide 13 is covered with an infrared reflective film. The infrared reflective film may be made of gold, silver, aluminum, or a dielectric multilayer membrane. Further, if necessary, a protective film made of silicon oxide or silicon nitride, for example, is formed on the infrared reflective film to prevent corrosion of the metal film of the infrared reflective film.

As shown by the bold arrows in FIG. 3B, the light guide 13 repetitively reflects, on its inner infrared reflective film, infrared rays entering the light guide 13 from the light source 2 and delivers the infrared rays to the light receiver 4. To the light receiving surface of the light receiver 4, the optical filter 3 is attached. By repetitively reflecting infrared rays emitted by the light source 2 on the infrared reflective film, the light guide 13 of the casing body 11 serves as an optical path that guides part of the reflected light to the light receiver 4 via the optical filter 3. With the infrared rays guided from the light source 2 to the light receiver 4, gas detection is performed.

The infrared reflective film is formed on the inner surfaces of the half-pipe parts 118A and 118B of the upper and lower parts 110A and 110B of the casing body 11 by plating (resin plating), sputtering, or vacuum deposition, for example. The infrared reflective film improves reflectivity and increases the amount of light reaching the light receiver 4.

As shown in FIG. 1, the gas intake ports 111 and 112 are formed as entrances of air inlets in the upper part 110A of the casing body 11. Further, as shown in FIG. 3A, gas introduction ports 131 and 132 are formed as exits of the air inlets in the inner wall of the curved parts of the light guide 13. The gases introduced through the gas intake ports 111 and 112 enter the inner space of the casing body 11 and are then introduced inside the light guide 13 via the gas introduction ports 131 and 132. The gas introduction ports 131 and 132 are formed such that, in a top view, the line connecting the center of the gas intake port 111/112 and the center of the gas introduction port 131/132 (the dashed line C in FIG. 3A) is at an angle of 45 degrees to the x axis.

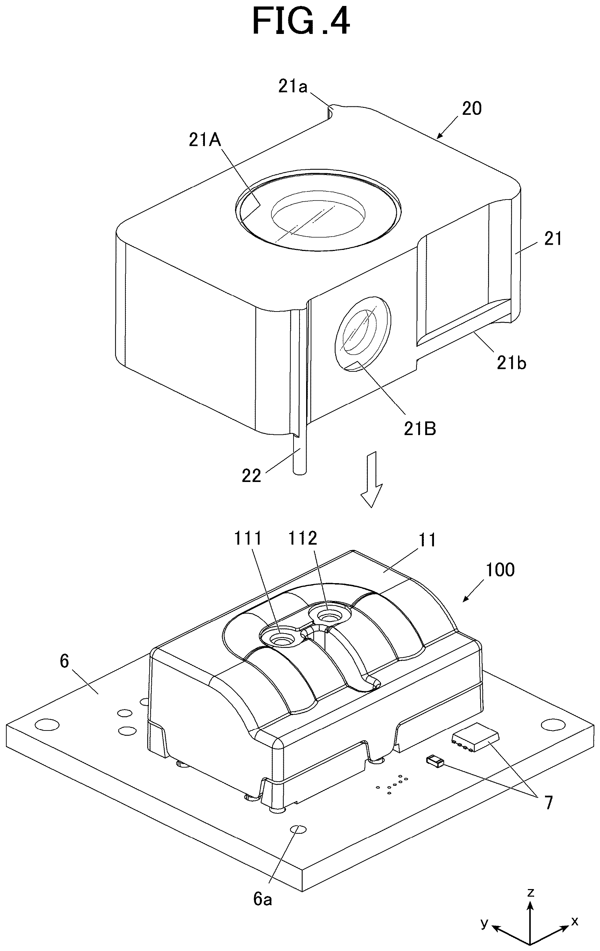

Next, the embodiment of the protective cover that protects the above-described gas sensor 10 from dust and droplets will be described with reference to FIG. 4 to FIG. 7.

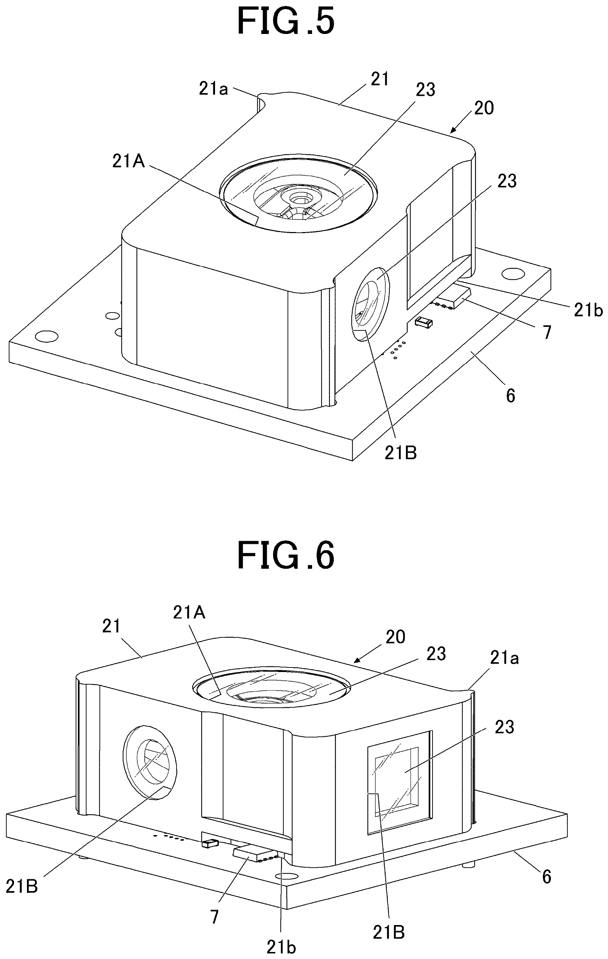

The protective cover 20 in the embodiment has a box shape, and one side (bottom side, bottom face) of the box-shaped protective cover 20 is open. As shown in FIG. 4, the protective cover 20 is mounted to cover the gas sensor 10 mounted on the board 6. Specifically, two fitting pieces 22 projecting downward are formed at opposite corners of the opening edge of the box-shaped body 21 of the protective cover 20. In FIG. 4, one of the two fitting pieces 22 is hidden and invisible. The fitting pieces 22 are inserted into fitting holes 6a (e.g., through holes) formed on the board 6. The fitting holes 6a are formed beforehand near the area where the gas sensor 10 is mounted. The portions of the fitting pieces 22 protruding from the bottom surface of the board 6 are caulked. Thus, the protective cover 20 is joined to the board 6 in the state of covering the gas sensor 10, as shown in FIG. 5. In this description, the gas sensor 10 covered by the protective cover 20 is referred to as the optical gas sensor device.

The structure for fitting the fitting pieces 22 to the board is not limited to the fitting holes 6a as through holes. For another example, grooves or counterbores may be formed in the board, and the ends of the fitting pieces 22 may be inserted therein and fixed with adhesive.

On the top surface of the board 6, electronic components (including elements) that constitute signal processing circuits and so forth are mounted. The gas sensor 10 is mounted on the top surface of the board 6 to cover the upper area of part of the electronic components. At predetermined positions on the board 6, non-illustrated pin holes are formed. Into the pin holes, the positioning pins 14 provided on the bottom surface of the casing body 11 of the gas sensor 10 can be inserted. When the positioning pins 14 are inserted into the pin holes on the board 6, the gas sensor 10 is mounted at a predetermined position on the board 6.

The board 6 is a printed circuit board (PCB) made of a glass epoxy resin plate or the like on which conductor wiring is printed.

The protective cover 20 may be made of synthetic resin or metal. When the protective cover 20 is made of synthetic resin, the tip ends of the fitting pieces 22 are deformed by thermal caulking. When the protective cover 20 is made of metal, the tip ends of the fitting pieces 22 are bent by 90 degrees by bending processing. In the example shown in FIG. 4, the fitting pieces 22 are formed at two opposite corners among the four corners of the box-shaped body 21. For another example, the fitting pieces 22 may be formed at all the four corners.

Although the protective cover 20 is not limited to a specific size, the protective cover 20 has such a size that forms a gap of 1 millimeter or less between four lateral walls except the top wall of the box-shaped body 21 and the corresponding four lateral surfaces of the gas sensor 10.

At least on the surface of the protective cover 20 (the top surface of the box-shaped body 21 in FIG. 4) corresponding to the surface having the gas intake ports 111 and 112 of the casing body 11 of the gas sensor 10, an opening 21A is formed. The area of the opening 21A is greater than the area of the gas intake ports 111 and 112. Further, in this embodiment, an opening 21B is formed on the front surface of the box-shaped body 21 (the surface at the side of the light source 2 and the light receiver 4) to improve the flow of the detection target gas. These openings 21A and 21B are each provided with a filter 23 that prevents entrance of dust and droplets. The filter 23 consists of a metal mesh, for example. In this embodiment, the peripheral edge of the filter 23 is joined and fixed to the inner wall of the box-shaped body 21 by adhesive.

The shapes of the openings 21A and 21B formed in the protective cover 20 are not limited to the round shape as shown in FIG. 4. The openings 21A and 21B may have any desired shapes, such as a rectangular or oval shape. The surfaces having the openings 21A and 21B are not limited to the top and front surfaces. The openings may be formed on three surfaces as shown in FIG. 6 or on all the five surfaces.

The means for fixing the filter 23 to the protective cover 20 is not limited to adhesive. The filter 23 can be fixed by other means, such as screws or fitting. The shape of the filter 23 is not limited to the shape corresponding to the shape of the protective cover 20 or the openings 21A and 21B. The filter 23 can have any desired shape. For example, rectangular filters can reduce the loss of edge materials as compared with round filters in cutting out the filters from materials.

Further, adhesive may be applied between the opening edge of the lower end of the lateral walls of the box-shaped body 21 and the board 6; or a thin-film sealing material having a rectangular ring shape may be interposed between the lower end surface of the lateral walls of the box-shaped body 21 and the top surface of the board 6. Thus, dust-proof and waterproof properties can be enhanced according to the ingress protection (IP) ratings required by customers. The sealing material may be joined to the opening edge of the box-shaped body 21 beforehand by adhesive, for example.

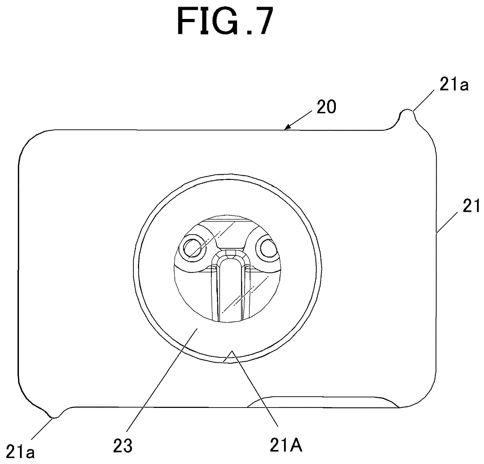

As shown in FIG. 7, the protective cover 20 of this embodiment has bulging portions 21a that extend in the up-down direction (the direction perpendicular to the paper surface of FIG. 7) at opposite two corners among the four corners of the box-shaped body 21. At the bulging portions 21a, the lateral walls of the box-shaped body 21 bulge outward. The fitting pieces 22 are formed at the bottom surfaces of the respective bulging portions 21a. The bulging portions 21a are formed to deal with the positional restriction of the fitting holes 6a on the board 6 with respect to the electronic components mounted on the board 6. The configuration of the protective cover 20 is not limited thereto. A casing without the bulging portions 21a is also adoptable.

The bulging portions 21a may be actively adopted to the structure of the protective cover 20. For example, assume that there are gas sensors 10 (products) having slightly different sizes as protection targets. In such a case, the positions of the fitting holes 6a on the board 6 are determined based on the gas sensor having the largest size; the protective cover corresponding to the largest gas sensor does not have the bulging portions 21a or has small bulging portions 21a on the lateral walls of the box-shaped body 21; and at the bottom surface of the protective cover, the fitting pieces 22 are formed. On the other hand, the protective cover corresponding to a smaller gas sensor has the bulging portions 21a having a relatively great protrusion amount on the lateral walls of the box-shaped body 21; and at the bottom surfaces of the bulging portions 21a, the fitting pieces 22 are formed.

Thus, in attaching the protective covers suitable for the respective gas sensors having different sizes, a drilling device for forming the fitting holes 6a in the board 6 and a caulking device for calking the tip ends of the fitting pieces 22 can be commonly used for multiple gas sensors having different sizes. This can reduce manufacturing costs.

As shown in FIG. 4, on the board 6, the gas sensor 10 as the protection target of the protective cover 20 in this embodiment is mounted, and the electronic components 7 are mounted near the gas sensor 10. If the surface of the box-shaped body 21 does not have a projection, recess, or notch, the lower part of the box-shaped body 21 of the protective cover 20 mounted on the board 6 may be in contact with the electronic components 7 near the gas sensor 10 mounted on the board 6. To deal with this, the protective cover 20 of this embodiment has a recess 21b at the bottom end of the front surface of the box-shaped body 21. The recess 21b is for preventing the protective cover 20 from being in contact with the electronic components 7.

With such a recess 21b, the protective cover 20 can be mounted on the board 6 on which the gas sensor 10 in operation and the electronic components 7 have been closely mounted, without creating unnecessary gaps between the protective cover 20 and the board 6. Since there is no need to avoid the electronic components, the outer shape of the protective cover 20 can be downsized.

As described above, the protective cover 20 in this embodiment has the opening 21A provided with the filter 23. The protective cover 20 is mounted to cover the gas sensor 10 mounted on the board 6. Such a protective cover 20 can prevent entrance of foreign substances, such as dust and droplets, into the sensor, without inhibiting the inflow of the detection target gas. Thus, corrosion of the reflective film formed on the inner surface of the light guide 13 of the gas sensor 10 is prevented, and the product life is extended. Further, the protective cover protects the casing body 11 of the gas sensor 10 from external force, thereby preventing physical damage to the gas sensor 10. Further, not only the sensor but also the other electronic components can be protected from foreign substances such as droplets.

The protective cover 20 is mounted to cover the gas sensor 10 and protects the gas sensor 10 from dust and droplets, rather than adding dust and droplet protection functions to the gas sensor 10 itself. Therefore, dust and droplets can be prevented without changing the design of the casing body 11 of the gas sensor 10.

Further, the protective cover 20 of this embodiment can be attached to the board 6 by various methods, such as adhesive, caulking, and sealing material. Further, the types (characteristics) of filters provided to the openings 21A and 21B can be changed. By changing the cover attaching method and the filter type, the IP ratings required by customers can be easily achieved.

Further, the size, the number, and the positions of the openings 21A, 21B formed on the protective cover can be changed. Such a protective cover allows swift responses to customer requirements. Further, by preparing multiple types of protective covers, the IP ratings required by customers can be easily and quickly achieved. Further, the protective cover not only has dustproof and waterproof performance but also allows changes in the permeability of the detection target gas. Therefore, sensor characteristics, such as detection time and sensitivity, can be easily modified.

Although the present invention has been described in detail based on the embodiment, the invention is not limited to the above embodiment. For example, in the above embodiment, the protective cover 20 has such a size that forms a gap of 1 millimeter or less between the gas sensor 10 and the protective cover 20. However, the protective cover 20 may be formed to have a gap of 1 millimeter or greater between the gas sensor 10 and the protective cover 20, depending on the environment in which the sensor is installed.

In the above embodiment, the protective cover 20 has the opening 21A at the part (top wall) facing the gas intake ports 111 and 112 formed on the top surface of the gas sensor 10. However, the opening(s) may be intentionally formed only on a side surface(s) that does not face the gas intake ports 111 and 112. This can improve dustproof and waterproof performance.

In the above embodiment, the protective cover is used for the gas sensor having the rectangular casing body in a top view, as an example. However, the gas sensor to be protected is not limited to the one having the rectangular shape. The present invention is also applicable to a protective cover for a gas sensor having a circular shape in a top view.

## Claims

1. A protective cover (20) to be mounted to cover an optical gas sensor (10), the optical gas sensor including: a casing body (11) that has a hollow part (13) into which a detection target gas is introduced and a gas intake port (111, 112) that communicates with the hollow part; and a light source (2) and a light receiver (4) disposed to face each other with the hollow part in-between, the optical gas sensor being mounted on a board (6) and configured to detect a gas concentration inside the hollow part, based on a detection signal of the light receiver that receives light emitted by the light source, wherein:
the protective cover is box-shaped and has a wall,
one side of the box-shaped protective cover is open,
at least part of the wall has an opening (21A) provided with a filter (23), and
at an open edge of the one side, the protective cover has at least a pair of fitting pieces (22) to be fitted to fitting portions (6a) of the board.

2. The protective cover for an optical gas sensor according to claim 1, wherein:
the protective cover is for the optical gas sensor that includes the cuboid-shaped casing body having the gas intake port on a surface opposite the board,
the protective cover is cuboid-shaped corresponding to the cuboid-shaped gas sensor,
the protective cover has walls at five sides among six sides of the cuboid shape of the protective cover,
the remaining one side of the protective cover is open, and
the opening is formed on a wall that is to face the gas intake port of the optical gas sensor.

3. The protective cover for an optical gas sensor according to claim 2, wherein, in addition to the wall having the opening, one or more walls among the five walls of the protective cover has an opening (21B) provided with a filter (23).

4. The protective cover for an optical gas sensor according to any one of claims 1 to 3, wherein at the open edge of the one side of the protective cover, a recess (21b) is formed for preventing the protective cover from being in contact with an electronic component (7) to be mounted on the board.

5. An optical gas sensor device comprising:
the protective cover (20) according to any one of claims 1 to 4; and
an optical gas sensor (10) that includes: a casing body (11) that has a hollow part (13) into which a detection target gas is introduced and a gas intake port (111, 112) that communicates with the hollow part; and a light source (2) and a light receiver (4) disposed to face each other with the hollow part in-between, the optical gas sensor being mounted on a board (6) and configured to detect a gas concentration inside the hollow part, based on a detection signal of the light receiver that receives light emitted by the light source.
